# EUROPÄISCHE PATENTSCHRIFT

(11) **EP 2 860 130 B1**
(45) Veröffentlichungstag und Bekanntmachung des Hinweises auf die Patenterteilung: **06.01.2016**
(21) Anmeldenummer: 13188379.5
(22) Anmeldetag: 11.10.2013
(51) Int. Cl.: B65D 51/32, A61M 35/00

(54) **Sicherheitsflasche**
Safety bottle
Bouteille de sécurité

(43) Veröffentlichungstag der Anmeldung: 15.04.2015
(73) Patentinhaber: Infectopharm Arzneimittel und Consilium GmbH, 64646 Heppenheim (DE)
(72) Erfinder: Freikamp, Louise, 65287 Darmstadt (DE); Peintner, Iris, 64646 Heppenheim (DE); Dornseiff, Martin, 64625 Bensheim (DE)
(74) Vertreter: Fuchs Patentanwälte Partnerschaft mbB

(56) Entgegenhaltungen:
- EP-A1- 0 354 823
- EP-A2- 0 072 772
- DE-A1-102007 046 600
- DE-U1- 20 112 135
- US-A- 4 503 872

## Beschreibung

Die vorliegende Erfindung betrifft eine auslaufsichere und kindersicher verschlossene Sicherheitsflasche, die mit einer flüssigen Substanz befüllt ist. Die flüssige Substanz kann mit der erfindungsgemäßen Sicherheitsflasche anwenderfreundlich und gefahrlos mit einer geeigneten Auftragevorrichtung der Sicherheitsflasche entnommen und gezielt auf eine Fläche aufgetragen werden.

### Technischer Hintergrund:

Warzen sind kleine, scharf begrenzte, erhabene oder flache gutartige Geschwülste der oberen Hautschicht (Epidermis), die meist durch Viren ausgelöst werden und durch Kontakt- oder Schmierinfektion verbreitet werden. Je nach Ausprägung und Lokalisation unterscheidet man verschiedene Warzentypen, z.B. Vulgäre Warzen (Verrucae vulgares), Plantarwarze (Verrucae plantares), Feigwarzen (Condylomata acuminata) und Dellwarzen (Molluscum contagiosum).

Warzen treten bei Patienten jeden Alters einzeln oder in Gruppen auf und sind häufig an den Händen oder Füßen lokalisiert. Da die Immunabwehr von Kindern noch nicht so ausgereift ist, sind diese genauso wie immunsupprimierte Personen besonders häufig von Warzen betroffen.

Dellwarzen treten häufig im Kindes- oder Kleinkindesalter auf und werden, anders als die meisten Warzentypen, nicht durch das humane Papillomvirus (HPV) sondern durch das Molluscum-contagiosum-Virus verursacht. Übliche Therapieoptionen sind die chirurgische Entfernung der Dellwarzen (Kürettage), die Vereisung der Warzen (Kryotherapie) oder die Behandlung mit ätzenden Flüssigkeiten. Diese zerstören das infizierte Gewebe oder induzieren eine Entzündung, was zum Abheilen der Warze führt. Bekannt sind Behandlungen mit Säuren oder Laugen, insbesondere mit Salicylsäure, Milchsäure, Trichloressigsäure, Monochloressigsäure, Harnstoff, Silbernitrat oder Kalilauge.

Eine Hornschwiele oder Hautschwiele (fachsprachlich Tylom, Tylosis oder Tylositas), umgangssprachlich auch "Hornhaut" genannt, ist eine Vernarbung der Haut mit lokaler Verdickung des Stratum corneum (Hyperkeratose). Sie tritt als Resultat einer chronisch-traumatischen Dermatitis auf. Die Behandlung kann topisch mit Salicylsäure, Harnsäure oder auch anderen Säuren oder Laugen erfolgen.

Ein Hühnerauge (auch Klavus bzw. Clavus) ist eine durch chronischen Druck auf knochennahe Haut bedingte, umschriebene, meist sehr schmerzhafte Hornschwielenbildung (reaktive Keratinisierung) mit zentralem, in die Tiefe gerichtetem Sporn. Sporn bezeichnet in diesem Zusammenhang die harte, kegelförmige Hyperkeratose, deren Spitze nach innen gerichtet ist. Hühneraugen können in jedem Alter auftreten und müssen in den meisten Fällen behandelt werden. Dies kann durch Hühneraugenmesser oder chirurgische Eingriffe geschehen, bevorzugt ist aber auch hier die topische Behandlung mit ätzenden Flüssigkeiten.

Ohne an solche Annahmen gebunden zu sein, ist es plausibel, dass mit Säuren oder Laugen unabhängig davon, ob und wenn ja durch welches Virus eine Infektion vorliegt, Warzen, wie Dellwarzen und Feigwarzen, Hühneraugen oder Hornschwielen erfolgreich behandelt werden können. Derartige Anwendungen sind nicht auf Kinder beschränkt sondern auch für Erwachsene geeignet.

Die Behandlung von Kindern mit ätzenden Flüssigkeiten, wie beispielsweise einer Kalilauge, wird in der Regel von den Eltern im häuslichen Umfeld durchgeführt und ist im vorliegenden Fall bei Kindern ab einem Alter von 2 Jahren möglich. Dabei ist es wichtig, dass die flüssige Substanz mit einer Auftragevorrichtung, z.B. einem schmalen Spatel, punktgenau auf die zu behandelnde Hautstelle aufgetragen wird. Dies gilt insbesondere für Warzen, wie Dellwarzen.

Kommt jedoch eine ätzende Flüssigkeit, wie beispielsweise eine Kalilauge, mit gesunder Haut, mit Schleimhäuten oder gar den Augen in Kontakt, kann es zu Reizungen bis hin zu starken Verätzungen kommen.

Deshalb sind die Sicherheitsanforderungen an Applikatoren für ätzende Flüssigkeiten hoch, was insbesondere dann gilt, wenn Kinder damit behandelt werden sollen. Dies gilt auch für Applikatoren zum Auftragen von anderweitig gefährlichen, flüssigen Substanzen, von denen Gefahren wie mögliche Vergiftungen, irreversible Verfärbungen etc. ausgehen können.

Gerade für Substanzen, die als giftig oder sehr giftig eingestuft sind, wie z.B. Monochloressigsäure gilt, dass schon sehr kleine Mengen Schäden anrichten, sofern Kinder diese Substanz oral aufnehmen.

### Definitionen:

Unter flüssig wird gemäß dieser Erfindung bei Raumtemperatur flüssig verstanden.

Eine Substanz ist erfindungsgemäß eine Flüssigkeit, Lösung, Dispersion oder auch Emulsion, die ätzend ist, nämlich eine Säure oder Lauge. Eine Gefahr kann durch das Verschlucken der Substanz ausgehen, was insbesondere, wie oben gesagt, für die orale Aufnahme von giftigen oder sehr giftigen Substanzen bei Kindern zutreffend ist.

Die Auftragevorrichtung kann in verschiedenen Formen vorliegen, wie als unterschiedlich geformter Spatel oder Pinsel. Der Fachmann wird die Auftragevorrichtung den Eigenschaften der aufzutragenden flüssigen Substanz anpassen.

### Stand der Technik:

US 4 503 872 offenbart eine Sicherheitsflasche für Make-up gemäß dem Oberbegriff des Anspruchs 1.

Aus dem Stand der Technik sind Textmarker bekannt, die dem Prinzip folgen, deshalb auslaufsicher zu sein, weil die Füllhöhe der färbenden Flüssigkeit im Stift gering gehalten wird. Es ist jedoch nicht vorgesehen, dass die färbende Flüssigkeit dem Stift mit einem Applikator entnommen werden kann. Überhaupt ist das Flüssigkeitsreservoir eines Textmarkers grundsätzlich nicht zu öffnen.

Im Handel erhältlich sind Behälter für Kalilauge, wobei sich die Lösung in einer 10 ml Kunststoffflasche befindet. Bei der Auslieferung ist die Flasche mit einem Transportverschluss (nicht kindersicher) verschlossen. Bei der ersten Anwendung tauscht der Anwender den Transportverschluss gegen den beiliegenden kindersicheren Spatelverschluss.

Während der Anwendung ist die Flasche jedoch nicht verschlossen, so dass die Gefahr besteht, dass die offene Flasche zum Beispiel durch ein wehrhaftes Kind umgestoßen wird oder anderweitig in die Hände eines Kindes gelangt. Die in dieser Situation auslaufende ätzende Lösung könnte das Kind selbst oder die umstehenden Personen verletzen oder Gegenstände beschädigen.

Um die Produktsicherheit zu optimieren wurde die Packungsgröße des Produktes von 10 ml auf 2 ml reduziert, da 2 ml der Lösung für die Therapie eines Patienten in der Regel ausreichend sind und die auslaufende Flüssigkeitsmenge im Falle eines Verschüttungsunfalles deutlich geringer ist. Auch bei diesem handelsüblichen Produkt wird die Lösung in eine 10 ml Kunststoffflasche abgefüllt und mit dem Transportverschluss verschlossen. Der kindersichere Spatelverschluss wird vom Anwender bei der ersten Anwendung auf die Flasche aufgebracht. Als zusätzliche Sicherheitsmaßnahme soll die Faltschachtel während der Anwendung als Standfuß für die Flasche verwendet werden. Dies ist möglich, da die Faltschachtel an einer Seite einen perforierten Kreis besitzt, den der Anwender heraustrennt und die Flasche in den so entstandenen "Ständer" hineinstellen kann. Auf diese Weise wird die Standfläche deutlich vergrößert und die Flasche oberhalb ihres Schwerpunkts durch die Faltschachtel stabilisiert. Die Gefahr, dass die Flasche versehentlich umkippt oder umgestoßen wird ist somit reduziert. Sollte es trotzdem zu einem Verschüttungsunfall kommen, z.B. weil die Flasche trotz "Standfuß" umgestoßen oder die Faltschachtel nicht nach Vorgabe als Standfuß verwendet wurde, ist die austretende Flüssigkeitsmenge durch die reduzierte Packungsgröße kleiner.

Es besteht jedoch trotzdem eine gewisse Gefahr, dass es durch auslaufende Kalilauge zu Verletzungen oder Sachbeschädigungen kommt. Ferner ist es denkbar, dass der Anwender die Konstruktion "Standfuß" nicht nutzt, da er sie möglicherweise umständlich oder unnötig findet oder dessen Funktion nicht versteht.

Das Austauschen des Transportverschlusses durch den kindersicheren Spatelverschluss ist nicht besonders anwenderfreundlich und hat den Nachteil, dass das Produkt vor der ersten Anwendung nicht kindersicher verschlossen ist. Ferner ist die Herstellung des Produktes gemäß dem Stand der Technik wegen der zwei Verschlüsse aufwändig und teuer.

### Aufgabe der Erfindung:

Es soll eine Sicherheitsflasche bereitgestellt werden, die von Kindern nicht geöffnet werden kann und sogar in geöffnetem Zustand das Auslaufen einer flüssigen Substanz unmöglich macht. Der kindersichere Spatelverschluss soll so ausgestaltet sein, dass dieser die Flasche zum einen dicht und kindersicher verschließt und zum anderen für das punkgenaue Auftragen kleiner Flüssigkeitsmengen geeignet ist.

### Lösung der Aufgabe:

Die Aufgabe wird erfindungsgemäß gelöst durch eine mit flüssiger und ätzender Substanz (9, 10, 11) befüllte Sicherheitsflasche (1) zum sicheren Bevorraten und Auftragen der flüssigen Substanz, umfassend einen Flaschenhals (6), eine Abschlusskante (7) als Auflage für den Dichtungsring (8) und einen kindersicheren Verschluss mit einer Auβenkappe (3) und einer Innenkappe (4), wobei der kindersichere Verschluss mit einer Vorrichtung zum Auftragen der flüssigen Substanz (5) verbunden ist, und die Sicherheitsflasche (1) einen Schutzeinsatz in der Form eines konisches Rohrs (2) umfasst, das sich in Richtung des Flaschenbodens verjüngt und das ein unteres Ende (12) aufweist, wobei die Vorrichtung zum Auftragen der flüssigen Substanz (5) durch den Schutzeinsatz durchgeführt ist, wobei die flüssige Substanz in der Sicherheitsflasche (1) in einer solchen Menge vorliegt, dass unabhängig von der Ausrichtung der Sicherheitsflasche im Raum der Füllstand stets unterhalb des unteren Endes (12) des Schutzeinsatzes (2) ist.

### Detaillierte Beschreibung der Erfindung:

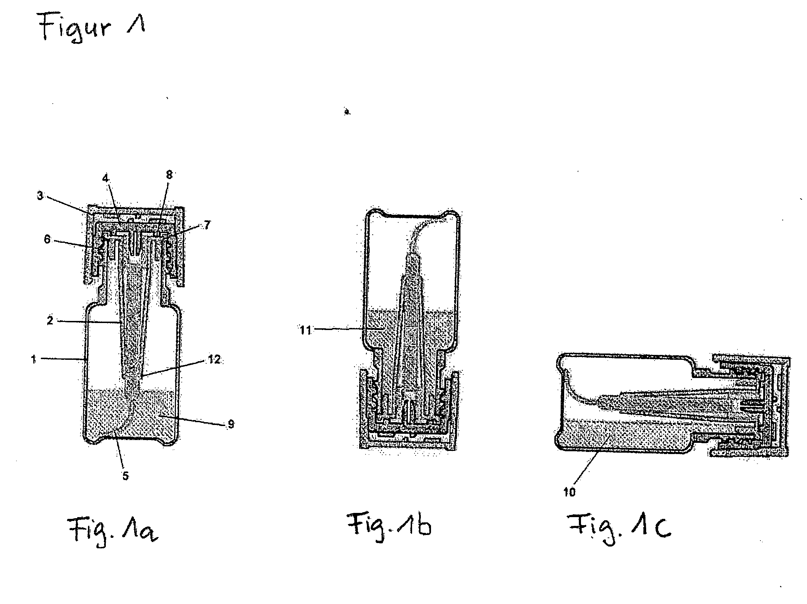

Die Abbildung zeigt die erfindungsgemäße Sicherheitsflasche in drei Ausrichtungen im Raum. Bei dem dort abgebildeten Spatel handelt es sich um eine mögliche Ausgestaltungsform einer Auftragevorrichtung und damit um eine besondere Ausführungsform der erfindungsgemäßen Sicherheitsflasche.

Die Sicherheitsflasche besteht vorzugsweise aus einem Material, das bei Raumtemperatur nicht verformbar ist, also weder plastisch noch elastisch ist, sofern eine Kraft darauf einwirkt, die durch einen Menschen mit bloßer Hand ausgeübt werden kann. Beispiele für solche Materialien sind Glas, Polypropylen oder Polyethylen, wobei es sich bei letzterem um HD (High-density) oder LD (Low-density) Polyethylen handeln kann. Bei der Wahl eines geeigneten LD Polyethylens wird der Fachmann die Bedingung zur Verformung berücksichtigen.

Das Volumen der Sicherheitsflasche kann bis zu 10 ml betragen, sofern mit der Sicherheitsflasche eine flüssige Substanz zur Behandlung von Warzen aufgetragen werden soll. Ein Flaschenvolumen von ca. 10 ml ist dabei eine bevorzugte Größe. Für andere, insbesondere großflächigere Anwendungen, wie die Behandlung von Hornhaut, kann die Sicherheitsflasche auch größer sein.

Das Füllvolumen der Sicherheitsflasche ist durch den Patentanspruch 1 definiert und ist demgemäß immer abhängig von dem maximalen Füllvolumen der Sicherheitsflasche selbst und der Länge des hineinragenden Rohrs. Für die bevorzugte Ausführungsform der Flasche mit einem Flaschenvolumen von 10 ml beträgt die Menge an flüssiger Substanz in der Flasche von 1 ml bis 4 ml, vorzugsweise von 2 ml bis 4 ml, weiter bevorzugt von 2,5 ml bis 3,5 ml, noch weiter bevorzugt von 2,8 ml bis 3,2 ml.

Gemäß einer bevorzugten Ausführungsform handelt es sich bei der flüssigen Substanz um eine wässrige Kaliumhydroxidlösung. Die Konzentration an KOH kann von 1 Gew.-% bis 10 Gew.-% betragen, vorzugsweise von 3 Gew.-% bis 8 Gew.-%, weiter bevorzugt von 4 Gew.-% bis 6 Gew.-%, und am meisten bevorzugt ca. 5 Gew.-% betragen. Die Konzentration kann jeweils ca. 1 Gew.-%, 2 Gew.-%, 3 Gew.-%, 4 Gew.-%, 5 Gew.-%, 6 Gew.-%, 7 Gew.-%, 8 Gew.-%, 9 Gew.-%, 10 Gew.-% betragen. Eine Kaliumhydroxidlösung ist bekannter Weise ätzend und kann zu Gewebeschädigungen führen, insbesondere wenn diese in Kontakt mit Schleimhäuten oder gar den Augen kommt.

Die Sicherheitsflasche kann wie folgt verwendet werden: Handelt es sich bei der flüssigen Substanz um wässrige Kaliumhydroxidlösung, wird die Sicherheitsflasche insbesondere zur Behandlung von Warzen, wie z.B. Dellwarzen (Molluscum contagiosum) oder Feigwarzen (Condylomata acuminata) eingesetzt, bevorzugt sofern diese bei Kindern oder Kleinkindern auftreten. Ganz bevorzugt ist die Behandlung von Dellwarzen, noch mehr bevorzugt von Dellwarzen bei Kindern. Die Sicherheitsfasche mit einer ätzenden oder keratolytischen Flüssigkeit kann auch zur Behandlung von Hühneraugen oder Hornhautschwielen verwendet werden, auch bei Erwachsenen.

Es kann sich bei der flüssigen Substanz auch um andere Säuren oder Laugen handeln, wie Salicylsäure, Milchsäure, Trichloressigsäure, Monochloressigsäure oder Harnstoff. Ist die Sicherheitsflasche damit befüllt, können damit auch alle Arten von Warzen behandelt werden und ebenfalls Hühneraugen und Hornhautschwielen.

Es wurde eingangs bereits beschrieben, dass die Sicherheitsflasche zur sicheren Verwahrung von verschiedenen Flüssigkeiten geeignet ist, von welchen eine Gefahr ausgehen kann. Die Verwendung der Flasche ist demgemäß flexibel. Eine lodlösung beispielsweise kann zur Desinfektion von oberflächlichen Wunden verwendet werden. Handelt es sich bei der flüssigen Substanz um Wirkstoffe, die bei oraler Einnahme toxisch sein können, wie Zytostatika, kann die Sicherheitsflasche beispielsweise zur Behandlung aktinischer Keratosen eingesetzt werden.

Eine bevorzugte Ausführungsform der Sicherheitsflasche und wie diese hergestellt wird, ist hiernach beschrieben:
Die flüssige Substanz, bevorzugt Kalilauge, weiter bevorzugt ca. 5%ige Kalilauge, wird in eine Kunststoffflasche gefüllt. Das Volumen der Flasche ist bevorzugt ca. 10 ml. Ein mögliches Material ist HDPE (High-density Polyethylen) und die Füllmenge der Kalilauge beträgt zwischen 2,8 und 3,2 ml.

In den Flaschenhals (6) wird ein Schutzeinsatz (2) eingesetzt. Es handelt sich dabei um ein konisches Rohr, vorzugsweise aus LDPE (Low-density Polyethylen). Dabei ist das Rohr dergestalt konisch, dass sich dieses in Richtung Flaschenboden verjüngt. Gemäß einer bevorzugten Ausführungsform ist der Durchmesser oben am Flaschenhals 4 bis 8 mm oder auch 6 bis 8 mm, denkbar auch 5 bis 7 mm und am unteren Ende (12) 1 mm bis 2 mm oder 1,5 mm bis 2,5 mm oder 2,5 mm bis 3,5 mm, bevorzugt 3 mm. Der Fachmann wird aus den genannten Bereichen geeignete obere und untere Grenzen wählen und diese frei miteinander kombinieren. Daraus ergibt sich ein Verhältnis von 2 zu 1 für oberen Durchmesser zu unterem Durchmesser. Das Verhältnis kann auch 3 zu 1, 2,5 zu 1, oder 1,5 zu 1 betragen. Der Schutzeinsatz (2) sitzt passgenau im Flaschenhals (6). Am oberen Ende hat der Abstreifer eine Abschlusskante (7), die auf dem Flaschenhals aufsitzt und eine plane Ringfläche darstellt, auf welche die ringförmige Dichtungskante des Spatelverschlusses (8) aufliegt, sobald der kindersichere Spatelverschluss aufgeschraubt ist. Die Dichtungskante ist gemäß bevorzugten Ausführungsformen in Form eines erhabenen Ringes vorhanden.

Gleich nach dem Abfüllen wird der kindersichere Verschluss, der die Außenkappe (3), Innenkappe (4) und Vorrichtung zum Auftragen der flüssigen Substanz (5), bevorzugt einem Spatel, umfasst, auf die Flasche aufgeschraubt.

Die Sicherheitsflasche weist ein größeres Volumen auf, als für die Füllmenge der flüssigen Substanz notwendig ist. Daher befindet sich der Flüssigkeitsspiegel stets im unteren Drittel der Flasche, egal ob die Flasche steht, liegt, oder auf dem Kopf gehalten wird. Dies wird auch aus der Abbildung ersichtlich, wobei (9), (10) und (11) die flüssige Substanz darstellen. Das konische Rohr des Schutzeinsatzes (2) ist so konzipiert, dass die in der Flasche enthaltene flüssige Substanz das untere Ende des Rohres (12) nie erreicht. Da die einzige Öffnung der Flasche das untere Ende des konischen Rohres des Schutzeinsatzes ist und der Flascheninhalt nicht durch dieses Rohr hindurch gelangen kann, ist sichergestellt, dass keine ätzende Lösung aus der Flasche auslaufen kann und zwar selbst dann wenn die Sicherheitsflasche während der Anwendung offen ist und umkippt oder in die Hände eines Kindes gerät.

Da die Sicherheitsflasche formstabil ist und vorzugsweise aus formstabilem HDPE besteht, kann sie bei der Anwendung nicht komprimiert werden. Somit ist es auch nicht möglich, die Flasche so weit zusammen zu drücken, dass der Flüssigkeitsspiegel bis auf die Höhe des Schutzeinsatzes ansteigt und herausgedrückt werden kann.

Die Entnahme der Lösung bei der Anwendung erfolgt vorzugsweise mit einem Spatel (5). Das Spatelende taucht durch den Schutzeinsatz, d.h. das konische Rohr, in die Flüssigkeit ein und wird mit dieser benetzt. Der Schutzeinsatz dient beim Herausziehen des Spatels als Abstreifer und stellt sicher, dass überschüssige Flüssigkeit abgesteift wird. Die am Spatel befindliche Flüssigkeitsmenge reicht zum Betupfen von 1 bis 3 Dellwarzen. Anschließend muss der Spatel durch erneutes Eintauchen in die Flüssigkeit neu benetzt werden.

### Bezugszeichenliste

(1) Sicherheitsflasche
(2) Schutzeinsatz mit konischem Rohr
(3) Außenkappe
(4) Innenkappe
(5) Vorrichtung zum Auftragen der flüssigen Substanz
(6) Flaschenhals
(7) Abschlusskante
(8) ringförmige Dichtung der der Innenkappe
(9) flüssige Substanz
(10) flüssige Substanz
(11) flüssige Substanz
(12) unteres Ende des konischen Rohrs

## Patentansprüche

1. Mit flüssiger Substanz (9, 10, 11) befüllte Sicherheitsflasche (1) zum sicheren Bevorraten und Auftragen der flüssigen Substanz, umfassend einen Flaschenhals (6), eine Abschlusskante (7) als Auflage für einen Dichtungsring (8) und einen kindersicheren Verschluss mit einer Außenkappe (3) und einer Innenkappe (4), wobei der kindersichere Verschluss mit einer Vorrichtung zum Auftragen der flüssigen Substanz (5) verbunden ist, und die Sicherheitsflasche (1) einen Schutzeinsatz in der Form eines konisches Rohrs (2) umfasst, das sich in Richtung des Flaschenbodens verjüngt und das ein unteres Ende (12) aufweist, wobei die Vorrichtung zum Auftragen der flüssigen Substanz (5) durch den Schutzeinsatz durchgeführt ist, wobei die flüssige Substanz in der Sicherheitsflasche (1) in einer solchen Menge vorliegt, dass unabhängig von der Ausrichtung der Sicherheitsflasche im Raum der Füllstand stets unterhalb des unteren Endes (12) des Schutzeinsatzes (2) ist,
**dadurch gekennzeichnet, dass**
es sich bei der flüssigen Substanz um eine ätzende Substanz, nämlich um eine Säure oder Lauge handelt.

2. Sicherheitsflasche (1) nach Anspruch 1, wobei der Schutzeinsatz mit konischem Rohr (2) am oberen Ende einen Durchmesser von 4 bis 8mm und am unteren Ende einen Durchmesser von 1 bis 3 mm aufweist.

3. Sicherheitsflasche (1) nach Anspruch 1 oder 2, wobei das maximale Flaschenvolumen bis zu 10 ml beträgt.

4. Sicherheitsflasche (1) nach einem der vorhergehenden Ansprüche, wobei die Menge an flüssiger Substanz von 2,8 bis 3,2 ml beträgt.

5. Sicherheitsflasche (1) nach einem der vorhergehenden Ansprüche, wobei das Material der Sicherheitsflasche ausgewählt ist aus Glas, Polyethylen oder Polypropylen.

6. Sicherheitsflasche (1) nach einem der vorhergehenden Ansprüche, wobei die flüssige Substanz eine Lauge ist, welche Lauge eine wässrige Lösung von Kaliumhydroxid (KOH) ist.

7. Sicherheitsflasche (1) nach Anspruch 6, wobei die Konzentration der wässrigen Lösung von KOH von 1 bis 10 Gew.-% beträgt.

8. Sicherheitsflasche (1) nach einem der Ansprüche 1 bis 7, die mit einer sauren oder basischen Lösung befüllt ist, zur Verwendung in einem Verfahren zur Behandlung von Warzen, Hühneraugen oder Hornhautschwielen.

9. Sicherheitsflasche nach Anspruch 8, wobei es sich bei der Lauge um Kalilauge handelt.

10. Sicherheitsflasche nach Anspruch 8 oder 9, wobei es sich bei den Warzen um Dellwarzen handelt.

## Claims

1. A safety bottle (1) filled with liquid substance (9), (10), (11) for safe storing and applying of the liquid substance, comprising a bottle neck (6), a finishing edge (7) as a bed for a sealing ring (8) and a childproof closure with an outer cap (3) and an inner cap (4), wherein the childproof closure is connected with a facility for applying the liquid substance (5) and the safety bottle (1) comprises a protective element in the form of a conical tube (2) which into the direction of the bottom of the bottle is tapered and comprises a lower end (12), wherein the facility for applying the liquid substance (5) passes through the protective element, wherein the liquid substance in the safety bottle (1) is present in such an amount that independent of the orientation of the safety bottle in the space the filling level is always below the lower end (12) of the protective element (2),
**characterized in that**
the liquid substance is an etching substance, namely an acid or a base.

2. The safety bottle (1) according to claim 1, wherein the protective element with conical tube (2) has a diameter at the upper end of 4 to 8 mm and a diameter at the lower end of 1 to 3 mm.

3. The safety bottle (1) according to claim 1 or 2, wherein the maximum volume of the bottle is up to 10 ml.

4. The safety bottle (1) according to one of the preceding claims, wherein the amount of liquid substance is 2.8 to 3.2 ml.

5. The safety bottle (1) according to one of the preceding claims, wherein the material of the safety bottle is selected from glass, polyethylene or polypropylene.

6. The safety bottle (1) according to one of the preceding claims, wherein the liquid substance is a base, wherein the base is an aqueous solution of potassium hydroxide (KOH).

7. The safety bottle (1) according to claim 6, wherein the concentration of the aqueous solution of KOH is 1 to 10 % by weight.

8. The safety bottle (1) according to one of claims 1 to 7, which is filled with an acidic or alkaline solution, for use in a method of treating warts, corns or horny skin.

9. The safety bottle according to claim 8, wherein the base is potassium hydroxide solution.

10. The safety bottle according to claim 8 or 9, wherein the warts are water warts (molluscum contagiosum).

## Revendications

1. Bouteille de sécurité (1) remplie d'une substance liquide (9, 10, 11) pour le stockage et l'application sûrs de la substance liquide, comprenant un goulot de bouteille (6), un bord terminal (7) en guise d'appui pour un anneau étanche (8) et une fermeture de sécurité pour enfants avec un bouchon extérieur (3) et un bouchon intérieur (4), la fermeture de sécurité pour enfants étant reliée à un dispositif pour l'application de la substance liquide (5), et le goulot de sécurité (1) comportant un insert de protection sous la forme d'un tube conique (2) qui se rétrécit en direction du fond de bouteille et qui présente une extrémité inférieure (12), le dispositif étant réalisé pour l'application de la substance liquide (5) par l'insert de protection, la substance liquide dans la bouteille de sécurité (1) se présentant dans une telle quantité qu'indépendamment de l'orientation de la bouteille de sécurité dans l'espace, le niveau est toujours sous l'extrémité inférieure (12) de l'insert de protection (2),
**caractérisée en ce**
**que** la substance liquide est une substance corrosive, à savoir un acide ou une lessive.

2. Bouteille de sécurité (1) selon la revendication 1, l'insert de protection avec un tube conique (2) sur l'extrémité supérieure présentant un diamètre de 4 à 8 mm et sur l'extrémité inférieure un diamètre de 1 à 3 mm.

3. Bouteille de sécurité (1) selon la revendication 1 ou 2, le volume de bouteille maximal s'élevant à 10 ml au maximum.

4. Bouteille de sécurité (1) selon l'une quelconque des revendications précédentes, la quantité de substance liquide étant comprise entre 2,8 et 3,2 ml.

5. Bouteille de sécurité (1) selon l'une quelconque des revendications précédentes, le matériau de la bouteille de sécurité étant sélectionné parmi le verre, le polyéthylène ou le polypropylène.

6. Bouteille de sécurité (1) selon l'une quelconque des revendications précédentes, la substance liquide étant une lessive qui est une solution aqueuse d'hydroxyde de potassium (KOH).

7. Bouteille de sécurité (1) selon la revendication 6, la concentration de la solution aqueuse de KOH étant comprise entre 1 et 10 % en poids.

8. Bouteille de sécurité (1) selon l'une quelconque des revendications 1 à 7, qui est remplie d'une solution acide ou basique pour l'utilisation dans un procédé de traitement de verrues, cors ou callosités.

9. Bouteille de sécurité selon la revendication 8, la lessive étant une lessive de potasse.

10. Bouteille de sécurité selon la revendication 8 ou 9, les verrues étant un *molluscum contagiosum.*
